(19) Europäisches Patentamt
European Patent Office
Office européen des brevets

(11) EP 3 434 766 A1

(12) EUROPEAN PATENT APPLICATION
published in accordance with Art. 153(4) EPC

(43) Date of publication:
30.01.2019 Bulletin 2019/05

(51) Int Cl.:
C12N 15/09 (2006.01)          A61P 27/02 (2006.01)
C12Q 1/68 (2018.01)

(21) Application number: 17770393.1

(22) Date of filing: 24.03.2017

(86) International application number:
PCT/JP2017/011934

(87) International publication number:
WO 2017/164353 (28.09.2017 Gazette 2017/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 25.03.2016 JP 2016061839

(71) Applicant: Santen Pharmaceutical Co., Ltd.
Higashiyodogawa-ku
Osaka-shi
Osaka 533-8651 (JP)

(72) Inventor: MATSUSHITA, Tokiyoshi
Ikoma-shi
Nara 630-0101 (JP)

(74) Representative: Müller-Boré & Partner
Patentanwälte PartG mbB
Friedenheimer Brücke 21
80639 München (DE)

(54) DETECTION METHOD AND DETECTION KIT FOR DETECTING DRY AGE-RELATED MACULAR DEGENERATION

(57) The object of the present invention is to provide a method, using blood as a sample, for accurately and conveniently measuring Alu RNA, which is a diagnosis marker for atrophic age-related macular degeneration and a target of a therapeutic agent for atrophic age-related macular degeneration.

According to the present invention, a method is provided for measuring an Alu RNA level in blood, comprising the following steps:

(1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA, and

(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product.

Fig. 1

**Description**

Technical Field

The present invention relates to a test kit for atrophic age-related macular degeneration and a test method for atrophic age-related macular degeneration using the kit.

Background Art

At present, age-related macular degeneration (AMD) is one of the major causative diseases of blindness in developed countries. Age-related macular degeneration is a disease of disordered retinal visual cells of the macula located at the fundus center caused by aging, and roughly classified into two types: atrophic age-related macular degeneration (atrophic AMD) and exudative age-related macular degeneration (exudative AMD). Atrophic age-related macular degeneration (atrophic AMD) is a disease associated with the geographic atrophy of photoreceptor cells and adjacent retinal pigment epithelial cells and choroidal capillaries and the accumulation of drusen containing a prophlogistic substance, and is said to account for 90% of age-related macular degeneration. On the other hand, exudative age-related macular degeneration (exudative AMD) is a disease wherein new blood vessels are formed from the choroid at the macula of an elderly person, causing bleeding or an exudative lesion under the retinal pigment epithelium or the retina to thereby finally form scar tissues.

A basic clinical condition of exudative AMD is the formation of choroidal new blood vessels, whereas atrophic AMD is known not to be associated with choroidal new blood vessels. Additionally, atrophic AMD has characteristics of slower progress than exudative AMD and thus a decline in vision is caused gradually, making it difficult to detect at an early stage. Symptoms of age-related macular degeneration include metamorphopsia and central scotomas, which cause a severe decline in vision when progress. Age-related macular degeneration is the No. 1 cause of acquired vison loss, and the number of patients tends to further increase from now on as the society ages, thereby aggressively promoting the elucidation of pathogenesis mechanism and the development of new drugs.

The method for treating age-related macular degeneration has been very advanced in recent years, but it fails to accomplish complete cure in most cases despite maintenance or improvement of the vision to a certain extent. The method for treating exudative AMD is mainly based on the occlusion of choroidal new blood vessels such as laser photocoagulation method, choroidal new blood vessel removal method, photodynamic therapy, and intravitreal injection of an anti-VEGF agent. In contrast, there is no effective method for treating atrophic AMD. Thus, the sooner establishment of a method for treating atrophic AMD is in demand.

In recent years, it is reported that atrophic AMD is most likely developed when Alu RNA accumulates in a RPE cell caused by the reduction of DICERI because the reduction of DICER1, which is a micro RNA (miRNA)-processing enzyme, and the accumulation of Alu RNA, which is an endogenous retroelement, are notably recognized in the retinal pigment epithelial (RPE) of an atrophic AMD human patient when compared with normal PRE, and PRE degeneration induced by the reduction of DICER1 is inhibited by antisense oligonucleotides of Alu RNA (Non-Patent Documents 1 and 2, and Patent Document 1, etc.). These reports indicate that Alu RNA can be a diagnosis marker for atrophic AMD, and the inhibition of an Alu RNA increase or the apoptosis signaling pathway caused by Alu RNA can be a target of a therapeutic agent for atrophic AMD, which are expected as new findings connecting to the development of therapeutic agents for atrophic AMD. In fact, a method for protecting RPE cells using siRNA targeting Alu RNA (Patent Document 1), a method for protecting RPE cells using inflammasome such as MyD88 as an Alu RNA inhibitor (Patent Document 2), and a method for treating retinal damages using an Alu RNA reverse transcription inhibitor (Patent Document 3), etc. have been reported based on these findings.

It is further reported that Alu RNA in blood, when measured, is also notably increased compared to healthy subjects of the same generation in addition to the RPE cell of AMD patients (Non Patent Document 3), but no specific measurement method is described.

Prior Art Documents

Patent Documents

Patent Document 1: Ambati et al., US 8,809,517 B2, Method of inhibiting Alu RNA and therapeutic uses thereof
Patent Document 2: Ambati et al., US 2014/0178309 A1, Protection of cells from alu-rna-induced degeneration and inhibitors for protecting cells
Patent Document 3: Ambati et al., US 2015/0038446 A1, Compositions and methods for treating retinal degradation

Non Patent Documents

Non-Patent Document 1: Hiroko Kaneko et al., "DICER1 deficit induces Alu RNA toxicity in age-related macular degeneration" Nature 471, 325-330 (2011)
Non-Patent Document 2: Valeria Tarallo et al., "DICER1 Loss and Alu RNA Induce Age-Related Macular Degeneration via the NLRP3 Inflammasome and MyD88" Cell Vol. 149, Issue 4, 847-859 (2012)
Non-Patent Document 3: B.Fowler et al., "Alu retrotransposon quantification in the retina and plasma: Mechanism-based risk assessment in age-related macular degeneration", Acta Ophthalmologica, Special Issue, Vol. 91, Issue Supplement s 252 (2013)

Summary of Invention

Problem to be Solved by the Invention

When Alu RNA, which can be a diagnosis marker for atrophic AMD or a stratification marker for atrophic AMD patients associated with the Alu RNA accumulation and a target of a therapeutic agent for atrophic AMD, is conveniently measured using blood as a sample, it is less of a burden for a subject and thus advantageous. However, Alu RNA, despite being a long non-coding RNA (Lnc RNA) molecule of about 300 bp, is generally unstable in blood in the form of free RNA, and it is thus easily conceivable that most of it is degraded to less than 80 to 100 bp, which is required by the existing real time PCR method, and further the Alu sequence is said to have 1 million copies present in the genome, extremely susceptible to the contamination of DNA compared with the PCR targeting common genes, and consequently causing most of the signals to be derived from the contaminated DNA, whereby the distinctive problems are found in the measurement of the Alu RNA level in blood. Further, contaminated DNA cannot be completely removed by a common DNase treatment. In fact, the study by the present inventors confirmed that, in some cases, even when a common DNase treatment is carried out, it is insufficient and signals derived from contaminated DNA do not disappear in the RT-PCR wherein the conventional forward primer and reverse primer designed based on the sequence present in Alu RNA (Alu RNA specific sequence) (Non Patent Document 1, etc.), or signals derived from contaminated DNA disappear when the treatment is carried out using a highly active DNase but RNA is degraded and RNA-derived signals cannot be obtained (see Reference Examples to be described later).
Accordingly, an object of the present invention is, in view of the above circumstances, to provide a method for accurately and conveniently measuring Alu RNA which has been degraded to a certain extent in blood.

Means for Solving the Problem

The present inventors extensively conducted studies to solve the above problems and found that, in the quantitative determination of Alu RNA in blood by the RT-PCR method, when a reverse transcription reaction is carried out, focusing on a region containing the Middle-A stretch sequence located on the middle part of the Alu RNA primary structure, using a reverse transcription primer that can anneal to such a region and having a universal tag sequence noncomplementary to Alu RNA at the 5' side, followed by carrying out PCR with the obtained cDNA as a template using a primer having a specific sequence to Alu RNA and a primer having a sequence complementary to the universal tag sequence, Alu RNA in blood can be measured without causing signals derived from contaminated DNA. The present invention was accomplished based on the finding.
More specifically, the present invention encompasses the following features.

[1] A test kit for atrophic age-related macular degeneration comprising the following primers (a) to (c):

(a) a reverse transcription primer having a poly dT sequence and a universal tag sequence at the 5' side of the poly dT sequence,
(b) a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine.
(c) a reverse primer consisting of a complementary sequence to the universal tag sequence of (a) or a partial sequence thereof.

[2] The kit according to [1], wherein the reverse transcription primer has a sequence comprising of 5 nucleotides or less a degenerate nucleotide at the 3' end of the poly dT sequence.

[3] The kit according to [2], wherein the sequence comprising a degenerate nucleotide is 5'-WG-3' (W represents A or T).

[4] The kit according to any of [1] to [3], wherein a length of the poly dT sequence is 10 to 40 nucleotides.

[5] The kit according to any of [1] to [4], wherein the reverse transcription primer is a primer consisting of a nucleotide sequence as set forth in SEQ ID NO: 3 or 4.

[6] The kit according to any of [1] to [5], wherein the forward primer is a primer consisting of a nucleotide sequence as set forth in SEQ ID NO: 5, or a nucleotide sequence of at least 15 nucleotides or more in the nucleotide sequence as set forth in SEQ ID NO: 5.

[7] The kit according to any of [1] to [5], wherein the forward primer is a primer consisting of a nucleotide sequence as set forth in SEQ ID NO: 6, or a nucleotide sequence of at least 15 nucleotides or more in the nucleotide sequence as set forth in SEQ ID NO: 6.

[8] A method for measuring an Alu RNA level in blood, comprising the following steps:

(1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA, and
(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product.

[9] A test method for atrophic age-related macular degeneration, comprising the following steps:

(1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA,
(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product, and
(3) comparing the measured Alu RNA level in blood of the subject to an Alu RNA level in blood of a control, and determining, based on the result, the severity of atrophic age-related macular degeneration in the subject.

[10] The method according to [8] or [9], further comprising a step of carrying out a polyadenylation reaction on RNA extracted from the blood sample collected from the subject, during or before the reverse transcription reaction in the step (1).

[11] The method according to any of [8] to [10], wherein the reverse transcription primer has a sequence of 5 nucleotides or less comprising a degenerate nucleotide at the 3' end of the poly dT sequence.

[12] The method according to [11], wherein the sequence comprising a degenerate nucleotide is 5'-WG-3' (W represents A or T).

[13] The method according to any of [8] to [12], wherein a length of the poly dT sequence is 10 to 40 nucleotides.

[14] The method according to any of [8] to [13], wherein the reverse transcription primer is a primer consisting of a nucleotide sequence as set forth in SEQ ID NO: 3 or 4.

[15] The method according to any of [8] to [14], wherein the forward primer is a primer consisting of a nucleotide sequence as set forth in SEQ ID NO: 5 or a nucleotide sequence of at least 15 nucleotides or more in the nucleotide sequence as set forth in SEQ ID NO: 5.

[16] The method according to any of [8] to [14], wherein the forward primer is a primer consisting of a nucleotide sequence as set forth in SEQ ID NO: 6, or a nucleotide sequence of at least 15 nucleotides or more in the nucleotide sequence as set forth in SEQ ID NO: 6.

[17] The method according to any of claims 8 to 16, wherein a length of the amplification product is 70 to 120 bp.

[18] A method for selecting, based on the Alu RNA level, a patient group for whom administration of an Alu RNA inhibitor is effective, wherein the method comprises measuring the Alu RNA level in blood of an atrophic age-related macular degeneration patient, using the method according to [8].

[19] A method for treating atrophic age-related macular degeneration, comprising measuring an Alu RNA level in blood of an atrophic age-related macular degeneration patient using the method according to [8], and administering an Alu RNA inhibitor to the atrophic age-related macular degeneration patient having a higher Alu RNA level than an Alu RNA level in blood of a healthy subject as a control.

[20] A therapeutic agent for atrophic age-related macular degeneration, comprising an Alu RNA inhibitor as an active ingredient, for administration to an atrophic age-related macular degeneration patient having a higher Alu RNA level in blood measured by the method according to [8] than an Alu RNA level in blood of a healthy subject as a control.

[21] An Alu RNA inhibitor for use in treating atrophic age-related macular degeneration targeting an atrophic age-related macular degeneration patient having a higher Alu RNA level in blood measured by the method according to [8] than an Alu RNA level in blood of a healthy subject as a control.

[22] Use of an Alu RNA inhibitor for treating atrophic age-related macular degeneration in an atrophic age-related macular degeneration patient having a higher Alu RNA level in blood measured by the method according to [8] than an Alu RNA level in blood of a healthy subject as a control.

[23] Use of an Alu RNA inhibitor for producing a therapeutic agent for atrophic age-related macular degeneration targeting an atrophic age-related macular degeneration patient having a higher Alu RNA level in blood measured by the method according to [8] than an Alu RNA level in blood of a healthy subject as a control.

The present invention claims the priority of Japanese Patent Application No. 2016-061839 filed on March 25, 2016 and the contents described in the specification of the patent application are included herein.

Effects of the Invention

According to the present invention, a kit and a method capable of accurately and conveniently measuring an Alu RNA level in blood are provided. Thus, when the kit of the present invention is used, the presence or absence of atrophic age-related macular degeneration and the severity of symptoms can be readily determined without imposing burdens on a subject.

Brief Description of Drawings

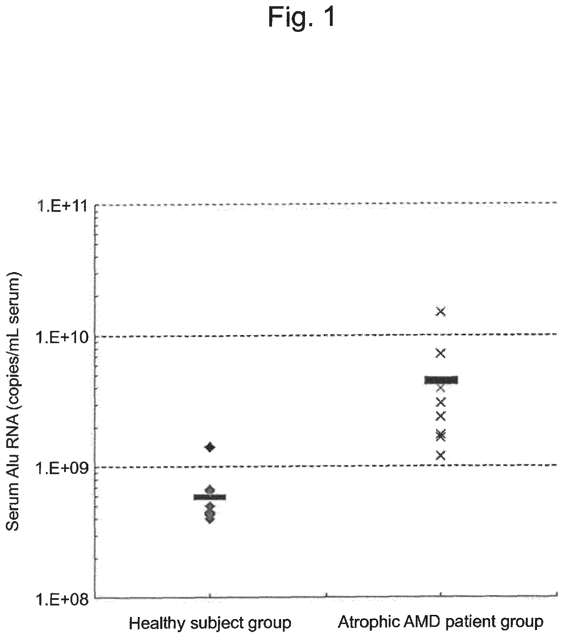

[Figure 1] shows the results on serum Alu RNA concentrations of healthy subject group and atrophic AMD patient group measured using the test kit for atrophic age-related macular degeneration of the present invention (Alu RNA (95-117) forward primer consisting of the nucleotide sequence as set forth in SEQ ID NO: 5 is used) (◊: serum Alu RNA concentration of healthy subject group, X: serum Alu RNA concentration of atrophic AMD patient group, bar: average value of each group, and dotted lines ($1.52 \times 10^9$ copies/mL serum) indicate 99.74% confidence interval of healthy subject group).

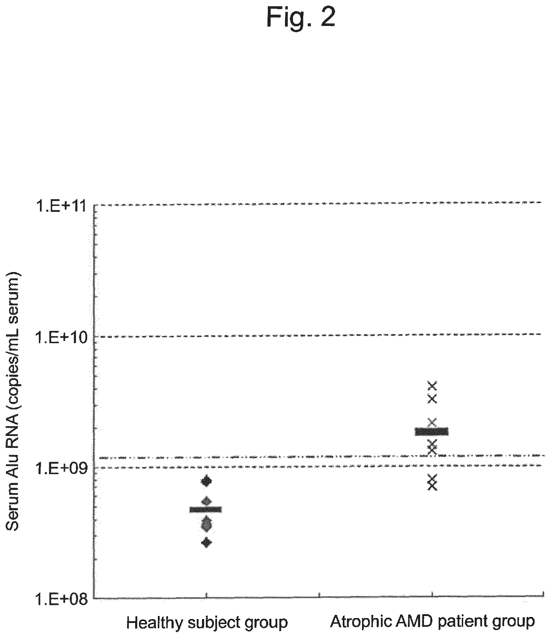

[Figure 2] shows the results on serum Alu RNA concentrations of healthy subject group and atrophic AMD patient group measured using the test kit for atrophic age-related macular degeneration of the present invention (Alu RNA 72-87) forward primer consisting of the nucleotide sequence as set forth in SEQ ID NO: 6 is used) (◊: serum Alu RNA concentration of healthy subject group, X: serum Alu RNA concentration of atrophic AMD patient group, bar: average value of each group, and dotted lines ($1.02 \times 10^9$ copies/mL serum) indicate 99.74% confidence interval of healthy subject group).

Embodiments for Carrying out the Invention

Hereinafter, the present invention is described in detail.

1. Test kit for atrophic age-related macular degeneration

The test kit for atrophic age-related macular degeneration (hereinafter referred to as "atrophic AMD") of the present invention comprises the following primers for measuring Alu RNA in a blood sample:

(a) a reverse transcription primer having a poly dT sequence and a universal tag sequence at the 5' side of the poly dT sequence,

(b) a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and

(c) a reverse primer consisting of a complementary sequence to the universal tag sequence of (a) or a partial sequence thereof.

Alu RNA is a RNA molecule having a chain length of about 300 bp and is classified under the long non-coding RNA (LncRNA) which does not encode a protein. Alu RNA has some subfamilies and, for example, Human Alu-Sq subfamily Consensus Sequence (Genbank accession No. HSU14573: SEQ ID NO: 1) and Human Alu-J subfamily Consensus Sequence (Genbank accession No. HSU14567: SEQ ID NO: 2) are known as the consensus sequence.

The "a reverse transcription primer having a poly dT sequence and a universal tag sequence at the 5' side of the poly dT sequence" of (a) constituting the kit of the present invention is a primer used for the reverse transcription reaction of Alu RNA by annealing to the Middle-A stretch part of Alu RNA, and the "universal tag sequence" refers to any unique sequence which is not present on the human genome and not complementary to an Alu RNA sequence. The universal tag sequence is utilized as the primer sequence for the PCR amplification of a reverse transcription product of Alu RNA (Alu cDNA), and thus the length thereof is preferably 15 to 40 nucleotides, more preferably 20 to 35 nucleotides, and further preferably 25 to 30 nucleotides. Further, such a reverse transcription primer may have a sequence of 5 nucleotides or less comprising a degenerate nucleotide at the 3' end thereof, and cDNA complementary to Alu RNA is synthesized by the reverse transcription reaction with the 3' end as the origin of synthesis. The sequence comprising a degenerate nucleotide is preferably 5'-WG-3' (W represents A or T).

The length of the poly dT sequence is not limited as long as the poly dT sequence can anneal to the Middle-A stretch part present in the nucleotide sequence at positions 121 to 136 in the nucleotide sequence (SEQ ID NO: 1) of Alu RNA, and has 5 nucleotides or more. For amplifying DNA fragments of about 70 bp to 120 bp, the length is preferably 10 to 40 nucleotides, and more preferably 20 to 30 nucleotides.

Examples of the reverse transcription primer of the present invention meeting the above conditions include 5'-GTG CAG GGT CCG AGG TTC ACT ATA GGT TTT TTT TTT TTT TTT TTT TTT TTA G-3' (SEQ ID NO: 3), or, 5'-GTG CAG GGT CCG AGG TTC ACT ATA GGT TTT TTT TTT TTT TTT TTT TTT TTT GT-3' (SEQ ID NO: 4) but not limited thereto.

The "a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine" of (b) constituting the kit of the present invention is a primer annealed to the Alu cDNA region synthesized by the above reverse transcription reaction and used for PCR-amplifying Alu cDNA, and examples include primers consisting of the nucleotide sequence as set forth in SEQ ID NO: 5 or SEQ ID NO: 6 below.

Alu RNA (95-117) forward primer:

5'-CAA CAT RGT GAA ACC CCG TCT CT-3' (R=G/A, SEQ ID NO: 5)

Alu RNA (72-87) forward primer:

5'-CAG GAG TTC GAG ACC A-3' (SEQ ID NO: 6)

Further, the above primer consisting of the nucleotide sequence as set forth in SEQ ID NO: 5 or SEQ ID NO: 6 may be a primer wherein modification is added to the nucleotide sequence within the range of having the substantially same function as the primer consisting of the nucleotide sequence as set forth in SEQ ID NO: 5 or SEQ ID NO: 6, and examples of such a modified primer include primers consisting of a nucleotide sequence comprising at least 15 contiguous nucleotides in the nucleotide sequence as set forth in SEQ ID NO: 5 or SEQ ID NO: 6. The modified primer may comprise at least 15 contiguous nucleotides in the nucleotide sequence as set forth in SEQ ID NO: 5 or SEQ ID NO: 6, but the total length is preferably 35 nucleotides or less, more preferably 30 nucleotides or less, and further preferably 27 nucleotides or less. The present site (3' end side or 5' end side) in the primer for the at least 15 contiguous nucleotides is also not limited. Additionally, examples of other modified primer include primers consisting of a nucleotide sequence having an identity of 90% or more, and preferably 95% or more, to the nucleotide sequence as set forth in SEQ ID NO: 5 or SEQ ID NO: 6. The modification includes deletion, substitution, insertion and addition, and the number of nucleotides to be modified is 3 or less, preferably 2 or less, and more preferably 1. Further, the addition of nucleotide may be at the 3' side or the 5' side.

The "a reverse primer consisting of a complementary sequence to the universal tag sequence or a partial sequence thereof" of (c) constituting the kit of the present invention is a primer used for PCR-amplification of Alu cDNA with the forward primer of (b) as a set, and the length thereof is not limited as long as annealable to the universal tag sequence and functional as the primer and maybe suitably determined in accordance with the universal tag sequence.

The above primers comprised in the kit of the present invention can be synthesized by a known method in the technical field concerned as the synthesis method of oligonucleotides such as phosphoramidite method or H-phosphonate method, using a commonly used DNA automatic synthesizer (for example, Model 394 manufactured by Applied Biosystems).

The kit of the present invention may comprise, as needed, a RNA extraction reagent, an agent for reverse transcription reaction (reverse transcription enzyme, dNTP, DTT, a buffer for reverse transcription reaction, RNase Inhibitor, and a tube for reaction, etc.), an agent for PCR amplification (DNA polymerase and a buffer for PCR amplification, etc.), a DNA molecule comprising a target sequence to be a positive control of PCR, a labeled substance, an agent for test such as a stain or a gel for electrophoresis, or an instruction.

2. Method for measuring Alu RNA in blood and test method for atrophic age-related macular degeneration

The method for measuring Alu RNA of the present invention comprises the following steps:

(1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA, and
(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product.

Further, the test method for atrophic age-related macular degeneration of the present invention carries out, after the above steps (1) and (2), step (3) for comparing the measured Alu RNA level in blood of the subject to an Alu RNA level in blood of a control, and determining the severity of atrophic age-related macular degeneration in the subject. The determination of severity of atrophic age-related macular degeneration herein includes the determination of presence or absence in suffering from atrophic age-related macular degeneration and the determination of progression level of atrophic age-related macular degeneration.

The reverse transcription primer used in step (1) and the forward primer and the reverse primer used in step (2) are as described above.

Hereinafter, each step is described.

Step (1):

In step (1), a reverse transcription reaction is carried out on RNA extracted from a blood sample collected from a subject as a template, using a reverse transcription primer having a poly dT sequence and a universal tag sequence at the 5' side of the poly dT sequence, to synthesize cDNA.

The subject to be a measurement target of an Alu RNA level is typically human but includes various mammals including laboratory animals such as mouse, rat, and rabbit.

Alu RNA present in blood is a free RNA molecule released in association with atrophic AMD and is long non-coding RNA. Alu RNA present in blood encompasses full-length Alu RNA, Alu RNA precursor present in the biosynthetic pathway, and RNA degradation product which is partially degraded while Alu RNA is released and present in blood.

Examples of the blood sample collected from the subject include a blood sample collected from a subject suspected of having atrophic AMD, and blood samples before and after administration of an Alu RNA inhibitor expected to be a therapeutic agent for atrophic AMD. Examples of the blood sample specifically include a plasma sample and a serum sample, but a plasma sample is preferable from the view point of convenience.

The extraction of RNA from a blood sample can be carried out by the guanidine-cesium chloride method or the acidic phenol method, etc. Additionally, commercial kits such as miRNeasy Serum/Plasma kit (Qiagen) and NucleoSpin miRNA plasma (Macherey Nagel) can also be used.

The reverse transcription reaction of the present step can suitably be carried out by a general technique using a common reverse transcription enzyme in the presence of dNTPs except that the above reverse transcription primer is used. For the reverse transcription enzyme, for examples, Moloney murine leukemia virus (M-MLV)-derived reverse transcription enzyme and avian myeloblastosis virus (AMV)-derived reverse transcription enzyme can be used. Commercial reagents and kits, etc. for the reverse transcription reaction can also be suitably utilized.

The reaction conditions for the reverse transcription reaction can be suitably determined in accordance with the reverse transcription enzyme used. For example, when an M-MLV-derived reverse transcription enzyme is used, the reaction can be carried out under the reaction conditions of maintaining 40 to 60 minutes at 42°C. Further, after the reverse transcription reaction, the reverse transcription enzyme may be deactivated. The deactivation of the reverse transcription enzyme can be carried out by heat treatment or chemical treatment.

Due to the above reverse transcription reaction, cDNA having a structure comprising the universal tag sequence, the poly dT sequence, and a DNA sequence complementary to Alu RNA, from the 5' side to the 3' side, is synthesized.

The method for measuring Alu RNA of the present invention further preferably comprises a step of carrying out a polyadenylation reaction on RNA extracted from a blood sample collected from a subject, during or before the reverse transcription reaction of the present step (1).

Step (2):

In step (2), the cDNA synthesized in step (1) is quantitatively determined and a level of the Alu RNA of interest contained in the RNA extracted from the blood sample is measured. For the quantitative determination of cDNA, the PCR method is preferable, and the real time PCR method is more preferable. Specifically, the PCR amplification is carried out using the primer set consisting of the above forward primer and the reverse primer, with the cDNA synthesized in step (1) as a template, and an Alu RNA level in blood is measured based on an amount of the amplification product. The length of a region to be PCR-amplified is 70 nucleotides or more, and preferably 70 to 120 nucleotides.

The PCR amplification is not particularly limited except that the primer set consisting of the above forward primer and the reverse primer is used, and may be carried out by a routine method. Specifically, a fragment comprising a specific gene sequence of the gene of interest is amplified by repeating a cycle including degeneration of template DNA, annealing of the primers to the template, and extension reaction of the primers using a thermostable enzyme (DNA polymerases such as Taq polymerase and *Thermus themophilis*-derived Tth DNA polymerase). For the composition of PCR reaction solution (template DNA amount, kind of buffer solution, primer concentration, type and concentration of DNA polymerase, and dNTP concentration, etc.) and the PCR reaction conditions (temperature cycle, number of cycles, etc.), conditions under which a PCR amplification product can be obtained with high sensitivity by PCR using the primer set can suitably be selected and determined by a person skilled in the art based on preliminary experiments, etc. The method for selecting suitable PCR reaction conditions based on Tm of the primer is well known by the technical field concerned, and can be carried out, for example, under the condition: pre-denaturation at 94°C for 5 minutes, 30 cycles with 1 cycle consisting of denaturation at 94°C for 50 seconds, annealing at 55°C for 50 seconds, extension at 72°C for 1 minute, followed by final extension at 72°C for 1 minute. However, the conditions illustrated here are only an example, and the enzyme to be used, reaction temperature, reaction time, and number of cycles can suitably be changed. Such a process of PCR operations can be carried out using a commercial PCR kit and PCR device by following the operating manual thereof. For the PCR device, for example, GeneAmp PCR System 9700 (manufactured by Applied Biosystems) can be used.

Real time PCR is a technique to quantitatively determine DNA to be a template based on the amplification factor by carrying out PCR using a real time PCR device and continuously measuring the amplification by PCR. This method commonly uses a device exclusively for real time PCR wherein a thermal cycler and a fluorescence spectrophotometer are integrated. First, PCR is carried out using a known amount of serially diluted DNA as the standard, based on which a calibration curve is created by plotting the number of cycles at which a certain amount of an amplification product is achieved in the region where the amplification occurs exponentially (threshold cycle; Ct value) as the vertical axis and a starting DNA amount as the horizontal axis. The reaction is also carried out under the same conditions for an unknown concentration of a sample to determine a Ct value, and from this value and the calibration curve, an amount of the DNA of interest in the sample can be measured. The real time PCR method includes, depending on the difference in the test method of a gene amplification product, a method where a fluorescent reagent is used and a method where a fluorescent-labeled probe is used. Examples of the method wherein a fluorescent reagent is used include intercalator method wherein an intercalator such as SYBR Green I, which is a fluorescence-emitting compound by binding to double stranded DNA with a primer set, is added to a PCR reaction system, and examples of the method wherein a fluorescent-labeled probe is used include TaqMan method, molecular beacon method, and cycling probe method wherein TaqMan probe, molecular beacon probe, and cycling probe are respectively used, but in the present invention the intercalator method is preferable. In the TaqMan method, PCR is carried out by adding to the PCR reaction system an oligonucleotide probe modified with a fluorescent substance (FAM, JOE, FITC, etc.) at the 5' end and a quencher substance (TAMRA, DABCYL, BHQ, etc.) at the 3' end. In the TaqMan method, the TaqMan probe specifically hybridizes to a template DNA in the amplification reaction by PCR under the conditions used for the polymerase extension reaction, and is degraded in association with the extension of DNA chain, i.e., the amplification of template DNA, thereby releasing the fluorescent substance to increase a fluorescence amount in the PCR solution. This increase in the fluorescence amount becomes an index of the template DNA amplification, whereby the state of amplification by PCR is conveniently tested in real time. The real time PCR method may be carried out based on a common method known by a person skilled in the art using a commercial real time PCR kit and a real time PCR device by following the operating manual attached thereto. For the commercial real time PCR device, for example, QuantiTect SYBR Green Master Mix (manufactured by Qiagen) and ABI Prism 7500 Fast Real-Time PCR System (manufactured by Applied Biosystems) can be used.

Step (3):

In step (3), an Alu RNA level in blood of a subject is compared to an Alu RNA level in a control sample. The control sample refers to a sample to be a comparison criterion, and may be, for example, either of a blood sample of a healthy subject (negative control) or a blood sample of an atrophic AMD patient (positive control). When an Alu RNA level in blood of a subject is significantly higher than an Alu RNA level of the negative control, the subject is determined to have suffered from atrophic AMD, and when is equivalent to an Alu RNA level of the negative control, the subject is determined to have not suffered from atrophic AMD. Further, when an Alu RNA level in blood of a subject is significantly lower than an Alu RNA level of the positive control, the subject can be determined to have not suffered from atrophic AMD, or that symptoms can be determined to have been ameliorated by treatment. A level of Alu RNA in blood of a subject may be measured as an absolute level by the comparison to an Alu RNA level of a control sample, but an absolute level of Alu RNA does not always need to be measured but the confirmation of relative relation with an Alu RNA level of a control sample is sufficient.

The increase (decrease) rate of an Alu RNA level in blood of a subject to an Alu RNA level in a control sample is commonly 2 times or more, preferably 3 times or more, more preferably 4 times or more, and most preferably 5 times or more, but when an increase (decrease) rate is 3 times or more, it is said to be reliable.

The "test" or "diagnosis" in the present invention typically means the determination of whether or not a subject suffers from atrophic AMD but is not limited thereto, and encompasses the determination of the severity of symptoms or the progression level of the disease, the determination of treatment effects on the disease, and the determination of whether or not there is a risk of recurrence of the disease after treatment. Further, the "test" or "diagnosis" can be replaced with "aid of test" or "aid of diagnosis" which does not encompass the diagnosis made by a doctor, and specifically refers to data acquisition for the "test" or "diagnosis".

3. Method for selecting patient group that will benefit from administration of Alu RNA inhibitor, and method for treating atrophic AMD

The present invention further provides a method for selecting a patient group for whom administration of an Alu RNA inhibitor is effective based on an Alu RNA level in blood of an atrophic AMD patient measured by the above measurement method, a method for treating atrophic AMD by administering the Alu RNA inhibitor to an atrophic AMD patient having a higher Alu RNA level in blood than an Alu RNA level in blood of a healthy subject as a control, and a therapeutic agent containing an Alu RNA inhibitor as an active ingredient to be administered to the atrophic AMD patient. Specifically, an atrophic age-related macular degeneration patient having a significantly high Alu RNA level in blood has the increase of Alu RNA in blood inhibited by the administration of the Alu RNA inhibitor and can be selected as a patient group in which clinical conditions are expected to be ameliorated or cured, and the treatment by the administration of the Alu RNA inhibitor can be carried out to the patient group. The "Alu RNA inhibitor" in the present invention means a low-molecular compound, a peptide, a protein, and a nucleic acid derivative, etc., which reduce an Alu RNA level in serum, for example by directly or indirectly inhibiting the transcription of Alu RNA. Examples specifically include an antisense nucleic acid to Alu RNA.

Examples

Hereinafter, the present invention is described in further detail in reference to Examples. However, these Examples do not intend to limit the present invention.

(Reference Example) Measurement of Alu RNA in serum by conventional method

(1) RNA purification

Serum cell-free RNA was purified from 600 $\mu$L each of 10 serum samples from healthy subjects and 8 serum samples from patients with atrophic age-related macular degeneration (hereinafter referred to as "atrophic AMD") associated with geographic atrophy (Bioreclamation IVT), using a NucleoSpin miRNA plasma (Macherey Nagel Gmbh & Co. KG) kit in accordance with the protocol (with general rDNaseI treatment) attached thereto. For the standardization of the purification operation among the samples and reverse transcription efficiency, $4.8 \times 10^8$ copies of nematode Cel-miR-39_1 spike-in control (Qiagen), which is an exogenous small RNA, was added to each sample after a lysis buffer was added.

(2) One-step real time PCR

The serum cell-free RNA samples obtained in (1) were subjected to one-step real time PCR to confirm the presence or absence of amplification signals caused by DNA contamination. The test was carried out under the following 4 conditions with the combinations of additional DNase treatment using a TURBO DNA-free kit containing a genetically engineered DNase (the activity with as much as 250 times the common DNase) (Ambion) or not and the reverse transcription enzyme was added or not. For positive controls, the following chemically synthesized partial sequences of Alu RNA were set.

Alu RNA (95-188) RNA positive controls:

5'-CAA CAU GGU GAA ACC CCG UCU CUA CUA AAA AUA CAA AAA UUA GCC GGG CGU GGU GGC GGG CGC CUG UAA UCC CAG CUA CUC GGG AGG CUG AGG C-3' (SEQ ID NO: 7)

Condition 1: No additional DNase treatment (-), reverse transcription enzyme added (+)
Condition 2: No additional DNase treatment (-), no reverse transcription enzyme added (-)
Condition 3: Additional DNase treatment (+), reverse transcription enzyme added (+)
Condition 4: Additional DNase treatment (+), no reverse transcription enzyme added (-)

The serum cell-free RNA samples and the positive controls were subjected to the additional DNase treatment in accordance with the protocol of TURBO DNA-free kit. For the samples with no additional DNase treatment, the equal amount of RNase-free water was added in place of TURBO DNase included in the kit. With the samples prepared above as the templates, a PCR reaction solution was prepared using the following primer set to Alu RNA consisting of Alu RNA (95-117) forward primer: 5'-CAA CAT RGT GAA ACC CCG TCT CT-3'(R=G/A, SEQ ID NO: 5) and Alu RNA (170-188) reverse primer: 5'-GCC TCA GCC TCC CGA GTA G-3'(SEQ ID NO: 8), and a QuantiTect SYBR Green RT-PCR kit (Qiagen). For the samples with no reverse transcription enzyme added, the equal amount of RNase-free water was added in place of the reverse transcription enzyme.

A plate containing the prepared PCR reaction solution was set in ABI Prism 7500 Fast Real-Time PCR System (Applied Biosystems) to carry out one-step real time PCR. PCR was carried out under the condition of a reverse transcription reaction at 50°C for 30 minutes, and, after a hot start of 95°C for 15 minutes, 40 cycles of 94°C for 15 seconds, 58°C for 30 seconds, and 72°C for 30 seconds. The number of copies in the PCR reaction solution was calculated by the calibration curve method using the following chemically synthesized known concentration Alu RNA (95-188) DNA standard for 1-step RT-PCR DNA as a sample for the calibration curve.

Alu RNA (95-188) DNA standard for 1-step RT-PCR:

5'-GCC TCA GCC TCC CGA GTA GCT GGG ATT ACA GGC GCC CGC CAC CAC GCC CGG CTA ATT TTT GTA TTT TTA GTA GAG ACG GGG TTT CAC TAT GTT G-3' (SEQ ID NO: 9)

The results are shown in the following Table 1. For the number of copies, a calibration curve lowest value of 1000 copies/well is defined as the detection limit, and below the detection limit is expressed as <1000.

[Table 1]

| Influence on Alu RNA amplification of additional DNase treatment or no treatment and reverse transcription enzyme added or not added | | | | |
|---|---|---|---|---|
| | Condition 1 Modified DNase (-)/ RT (+) | Condition 2 Modified DNase (-)/ RT (-) | Condition 3 Modified DNase (+)/ RT (+) | Condition 4 Modified DNase (+)/ RT (-) |
| Serum from Healthy subject | 11632000 | 11299000 | <1000 | <1000 |
| Serum from AMD patient | 1507240 | 1861800 | 4718.44 | <1000 |
| Positive control | 93531.4 | <1000 | 22891.8 | <1000 |
| | | | | (copies/well) |

As shown in Table 1, under the conditions (condition 2) of no additional DNase treatment and no reverse transcription enzyme added, amplification signals were recognized in PCR of the serum samples from both healthy subjects and atrophic AMD patients. In other words, it is suggested that the amplification signals are derived from the genomic DNA, which are not sufficiently removed by the rDNase I treatment included in the purification kit. Further, the amplification signals were also recognized in an about same amount under the conditions (condition 1) of no additional DNase treatment and the reverse transcription enzyme added, based on which it is considered that most of the RNA-derived signals are masked with the genomic DNA-derived signals. This is presumably because as many as 1 million copies of the Alu element are present on the human genome. On the other hand, the amplification signals disappeared in PCR of the serum samples from both healthy subjects and atrophic AMD patients under the conditions (condition 4) of the additional DNase treatment and no reverse transcription enzyme added, and it is thus conceivable that the influence of genomic DNA was removable by the additional DNase treatment, but under the conditions (condition 3) of the additional DNase treatment and the reverse transcription enzyme added, RNA-derived amplification signals were not recognized in some samples. As one of the reasons for this, deterioration of the RNA quality during the additional DNase treatment was suggested because the amplification signals also decreased by the additional DNase treatment in the synthetic Alu RNA as the positive control. Further, Alu RNA is degraded in the blood circulation or during the treatment process from blood collection to serum collection as RNA is generally unstable in blood, and it was thus also conceivable that the sequence having a 94-nucleotide length, which is the amplification target, was not maintained.

(Example 1) Measurement of serum Alu RNA by the present method

Serum cell-free RNA was purified from serum of healthy subjects and serum from atrophic AMD patients in the same manner as Reference Example. A reverse transcription reaction was carried out using the purified serum cell-free RNA, $2.5 \times 10^7$ copies of Cel-miR39 (Qiagen) as the reverse transcription control for calculating the reverse transcription efficiency, or $2.5 \times 10^7$ copies of 72 to 120 in the following Alu Sq consensus sequence as templates (when a reverse transcription efficiency is 100%, the number of template copies in the PCR reaction solution is $5 \times 10^5$ copies).
Alu RNA (72-120) RNA positive control:

5'-CAG GAG UUC GAG ACC AGC CUG GCC AAC AUG GUG AAA CCC CGU CUC UAC U-3' (SEQ ID NO: 10)

When the purified serum cell-free RNA was used as a template, the reverse transcription reaction was carried out for 60 minutes at 37°C with TAKARA Thermal Cycler Dice (Takara Bio Inc.) using M-MLV reverse transcription enzyme (NIPPON GENE CO., LTD.), Poly (A) polymerase (Takara Bio Inc.), and Alu RT primer (5'-GTG CAG GGT CCG AGG TTC ACT ATA GGT TTT TTT TTT TTT TTT TTT TTT TTA G-3':SEQ ID NO: 3), Alu J RT primer (5'-GTG CAG GGT CCG AGG TTC ACT ATA GGT TTT TTT TTT TTT TTT TTT TTT TTT GT-3':SEQ ID NO: 4) having a sequence which is not present in the human genome at the 5' region, a poly dT sequence at the middle part, and 2 nucleotides determining the annealing position at the 3' end.
Further, when Cel-miR39 (Qiagen) was used as a template, the reverse transcription reaction was carried out under the same condition as above except that Cel-miR39 RT primer (5'-GTG CAG GGT CCG AGG TTC ACT ATA GGT TTT TTT TTT TTT TTT TTT TTT TTC A-3':SEQ ID NO: 11) was used.

With the obtained cDNA as a template, real time PCR was carried out using ABI Prism 7500 Fast Real-Time PCR System (Applied Biosystems) to quantitatively determine serum Alu RNA and control Cel-miR-39.

The real time PCR reaction for quantitatively determining Alu RNA was carried out using the following Alu RNA (95-117) forward primer or Alu RNA (72-87) forward primer consisting of a sequence specific to Alu RNA and the following universal reverse primer having a complementary sequence at the 5' region of the above reverse transcription primer with QuantiTect SYBR Green Master Mix (Qiagen) under the following condition: initial denaturation at 95°C for 15 minutes, 40 cycles of 94°C for 15 seconds, 55°C for 30 seconds, and 70°C for 30 seconds.

Alu RNA (95-117) forward primer:

5'-CAA CAT RGT GAA ACC CCG TCT CT-3'(R=G/A, SEQ ID NO: 5)

Alu RNA (72-87) forward primer:

5'-CAG GAG TTC GAG ACC A-3' (SEQ ID NO: 6)

universal reverse primer:

5'-GTG CAG GGT CCG AGG T-3' (SEQ ID NO: 12)

The real time PCR reaction for quantitatively determining Cel-miR-39 was carried out under the same conditions as above except that a forward primer (5'-TCA CCG GGT GTA AAT CAG C-3': SEQ ID NO: 13) specific to Cel-miR39 was used as the forward primer.

Further, for the quantitative determination of both Alu RNA and Cel-miR-39, PCR was carried out with chemically synthesized known concentration DNA (Alu (72-120+50) DNA standard and Cel-miR39 DNA standard) as samples for the calibration curve, using the above set of primers respectively, and the number of copies in the PCR reaction solution was calculated from the created calibration curve. Note that the chemically synthesized Alu RNA (72-120) RNA positive control (SEQ ID NO: 10) and Cel-miR-39_1 spike-in control (Qiagen) described above were subjected to the reverse transcription reaction and the real time PCR reaction in the same manner as the test samples, and measured values were calculated from the calibration curve to thereby calculate a reverse transcription efficiency based on the theoretical value.

Alu (72-120+50) DNA standard:

5'-GTG CAG GGT CCG AGG TTC ACT ATA GGT TTT TTT TTT TTT TTT TTT TTT TTA GTA GAG ACG GGG TTT CAC CAT GTT GGC CAG GCT GGT CTC GAA CTC CTG-3' (SEQ ID NO: 14)

Cel-miR39 DNA standard:

5'-GTG CAG GGT CCG AGG TTC ACT ATA GGT TTT TTT TTT TTT TTT TTT TTT TTC AAG CTG ATT TAC ACC GGG TGA-3' (SEQ ID NO: 15)

$4.8 \times 10^8$ copies of Cel-miR-39 was added to 600 μL of serum as an internal standard, and thus Cel-miR-39 per mL of serum is $8 \times 10^8$ copies. Accordingly, the number of Alu RNA copies per mL of serum was calculated using the following formula (1) from the number of copies of Cel-miR-39 and Alu RNA in each of the PCR reaction solutions measured. The reverse transcription efficiency was calculated using the following formula (2).

Number of Alu RNA copies in 1 ml of serum =

$8 \times 10^8 \times$ (number of Alu RNA copies in PCR reaction solution/Alu RNA reverse transcription efficiency)/(number of Cel-miR-39 copies in PCR reaction solution/Cel-miR39 reverse transcription efficiency) (1)

Reverse transcription efficiency of each measurement target molecule (%) =

$100 \times$ (measured value of reverse transcription control calculated from calibration curve/500,000) (2)

wherein 500,000 indicates the theoretical number of template copies in the PCR reaction solution when a reverse transcription efficiency is 100%.)

Figure 1 shows the measurement results when the forward primer (SEQ ID NO: 5) to the nucleotide sequences 95 to 117 of Alu RNA was used, and Figure 2 shows the measurement results when the forward primer (SEQ ID NO: 6) to the nucleotide sequences 72 to 87 of Alu RNA was used.

As shown in Figure 1 and Figure 2, according to the method of the present invention, it was demonstrated that Alu RNA was detected with high sensitivity, and the serum Alu RNA concentration in AMD patients were higher than the serum Alu RNA concentration in healthy subjects whichever the forward primer was used. Further, for example, when the 99.74% confidence interval value of the healthy subject group shown in the figures is set as the cut-off value, a patient with aggravated Alu RNA can be selected, and the present invention is considered to be useful, for example, as a biomarker for selecting a patient population who can benefit from a drug.

Industrial Applicability

The present invention can be utilized in the production and development field of test drugs and therapeutic drugs for atrophic age-related macular degeneration.

All publications, patents, and patent applications cited herein are incorporated by reference.

**Claims**

1. A test kit for atrophic age-related macular degeneration comprising the following primers (a) to (c):

   (a) a reverse transcription primer having a poly dT sequence and a universal tag sequence at the 5' side of the poly dT sequence,
   (b) a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and
   (c) a reverse primer consisting of a complementary sequence to the universal tag sequence of (a) or a partial sequence thereof.

2. The kit according to claim 1, wherein the reverse transcription primer has a sequence of 5 nucleotides or less comprising a degenerate nucleotide at the 3' end of the poly dT sequence.

3. The kit according to claim 2, wherein the sequence comprising a degenerate nucleotide is 5'-WG-3' (W represents A or T).

4. The kit according to any of claims 1 to 3, wherein a length of the poly dT sequence is 10 to 40 nucleotides.

5. The kit according to any of claims 1 to 4, wherein the reverse transcription primer is a primer consisting of a nucleotide sequence as set forth in SEQ ID NO: 3 or 4.

6. The kit according to any of claims 1 to 5, wherein the forward primer is a primer consisting of a nucleotide sequence as set forth in SEQ ID NO: 5 or a nucleotide sequence of at least 15 nucleotides or more in the nucleotide sequence as set forth in SEQ ID NO: 5.

7. The kit according to any of claims 1 to 5, wherein the forward primer is a primer consisting of a nucleotide sequence as set forth in SEQ ID NO: 6, or a nucleotide sequence of at least 15 nucleotides or more in the nucleotide sequence as set forth in SEQ ID NO: 6.

8. A method for measuring an Alu RNA level in blood, comprising the following steps:

   (1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA, and

(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product.

9. A test method for atrophic age-related macular degeneration, comprising the following steps:

(1) carrying out a reverse transcription reaction on RNA extracted from a blood sample collected from a subject as a template, using a reverse transcription primer having a poly dT sequence and a universal tag sequence at the 5' side of the poly dT sequence, to synthesize cDNA,
(2) carrying out PCR amplification on the cDNA synthesized in step (1) as a template, using a forward primer consisting of a nucleotide sequence comprising at least 15 contiguous nucleotides from a nucleotide sequence consisting of nucleotides at positions 1 to 117 in the nucleotide sequence as set forth in SEQ ID NO: 1, wherein the uracil of the nucleotides is replaced by the thymine, and a reverse primer consisting of a complementary sequence to the universal tag sequence or a partial sequence thereof, and measuring an Alu RNA level in blood based on an amount of the amplification product, and
(3) comparing the measured Alu RNA level in blood of the subject to an Alu RNA level in blood of a control, and determining, based on the result, the severity of atrophic age-related macular degeneration in the subject.

10. The method according to claim 8 or 9, further comprising a step of carrying out a polyadenylation reaction on RNA extracted from the blood sample collected from the subject, during or before the reverse transcription reaction in the step (1).

11. The method according to any of claims 8 to 10, wherein the reverse transcription primer has a sequence of 5 nucleotides or less comprising a degenerate nucleotide at the 3' end of the poly dT sequence.

12. The method according to claim 11, wherein the sequence comprising a degenerate nucleotide is 5'-WG-3' (W represents A or T).

13. The method according to any of claims 8 to 12, wherein a length of the poly dT sequence is 10 to 40 nucleotides.

14. The method according to any of claims 8 to 13, wherein the reverse transcription primer is a primer consisting of a nucleotide sequence as set forth in SEQ ID NO: 3 or 4.

15. The method according to any of claims 8 to 14, wherein the forward primer is a primer consisting of a nucleotide sequence as set forth in SEQ ID NO: 5 or a nucleotide sequence of at least 15 nucleotides or more in the nucleotide sequence as set forth in SEQ ID NO: 5.

16. The method according to any of claims 8 to 14, wherein the forward primer is a primer consisting of a nucleotide sequence as set forth in SEQ ID NO: 6, or a nucleotide sequence of at least 15 nucleotides or more in the nucleotide sequence as set forth in SEQ ID NO: 6.

17. The method according to any of claims 8 to 16, wherein a length of the amplification product is 70 to 120 bp.

18. A method for selecting, based on the Alu RNA level, a patient group for whom administration of an Alu RNA inhibitor is effective, wherein the method comprises measuring the Alu RNA level in blood of an atrophic age-related macular degeneration patient, using the method according to claim 8.

19. A method for treating atrophic age-related macular degeneration, comprising measuring an Alu RNA level in blood of an atrophic age-related macular degeneration patient using the method according to claim 8, and administering an Alu RNA inhibitor to the atrophic age-related macular degeneration patient having a higher Alu RNA level than an Alu RNA level in blood of a healthy subject as a control.

20. A therapeutic agent for atrophic age-related macular degeneration, comprising an Alu RNA inhibitor as an active ingredient, for administration to an atrophic age-related macular degeneration patient having a higher Alu RNA level in blood measured by the method according to claim 8 than an Alu RNA level in blood of a healthy subject as a control.

21. An Alu RNA inhibitor for use in treating atrophic age-related macular degeneration targeting an atrophic age-related macular degeneration patient having a higher Alu RNA level in blood measured by the method according to claim 8 than an Alu RNA level in blood of a healthy subject as a control.

22. Use of an Alu RNA inhibitor for treating atrophic age-related macular degeneration in an atrophic age-related macular degeneration patient having a higher Alu RNA level in blood measured by the method according to claim 8 than an Alu RNA level in blood of a healthy subject as a control.

23. Use of an Alu RNA inhibitor for producing a therapeutic agent for atrophic age-related macular degeneration targeting an atrophic age-related macular degeneration patient having a higher Alu RNA level in blood measured by the method according to claim 8 than an Alu RNA level in blood of a healthy subject as a control.

# Fig. 1

# Fig. 2

## INTERNATIONAL SEARCH REPORT

| International application No. |
| --- |
| PCT/JP2017/011934 |

**A. CLASSIFICATION OF SUBJECT MATTER**
*C12N15/09*(2006.01)i, *A61P27/02*(2006.01)i, *C12Q1/68*(2006.01)i

According to International Patent Classification (IPC) or to both national classification and IPC

**B. FIELDS SEARCHED**

Minimum documentation searched (classification system followed by classification symbols)
C12N15/09, A61P27/02, C12Q1/68

Documentation searched other than minimum documentation to the extent that such documents are included in the fields searched
Jitsuyo Shinan Koho       1922-1996   Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)
JSTPlus/JMEDPlus/JST7580(JDreamIII), PubMed, CAplus/REGISTRY/MEDLINE/
EMBASE/BIOSIS/WPIDS(STN), CiNii, GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X<br>A | US 8809517 B2 (AMBATI Jayakrishna),<br>19 August 2014 (19.08.2014),<br>claims 1 to 4; column 26, lines 35 to 55<br>& US 2013/0197207 A1    & US 2014/0342357 A1<br>& WO 2011/153234 A2 | 20-23<br>1-19 |
| X<br>A | KANEKO H. et al., DICER1 deficit induces Alu<br>RNA toxicity in age-related macular<br>degeneration., NATURE, 2011, Vol 471, p.325-330,<br>abstract, Figs.2-5, METHODS, paragraph of<br>miRNA PCR | 20-23<br>1-19 |
| A | FOWLER B. et al, Alu retrotransposon<br>quantification in the retina and plasma:<br>Mechanism-based risk assessment in age-related<br>macular degeneration., Acta Ophthalmologica,<br>2013, Vol.91 Issue s252, T081, entire text | 1-23 |

☒ Further documents are listed in the continuation of Box C.　　☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 June 2017 (01.06.17) | 13 June 2017 (13.06.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

## INTERNATIONAL SEARCH REPORT

International application No.

PCT/JP2017/011934

C (Continuation).   DOCUMENTS CONSIDERED TO BE RELEVANT

| Category* | Citation of document, with indication, where appropriate, of the relevant passages | Relevant to claim No. |
|---|---|---|
| A | US 2010/0112644 A1  (KIM D.Y et al.),<br>06 May 2010 (06.05.2010),<br>abstract; paragraphs [0042] to [0053]; claim 1;<br>SEQ ID NOs 1 and 7<br>& US 2013/0122511 A1 | 1-23 |
| A | CN 103966327 A  (Xinxiang Medical University),<br>06 August 2014 (06.08.2014),<br>paragraphs [0005] to [0020]; examples<br>(Family: none) | 1-23 |
| A | WO 2010/103522 A1  (ROSETTA GENOMICS LTD.),<br>16 September 2010 (16.09.2010),<br>claim 1; page 19, lines 5 to 13<br>(Family: none) | 1-23 |
| A | Tzu-Huey Li et al., Differential stress<br>induction of individual Alu loci: implications<br>for transcription and retrotransposition.,<br>Gene, 2001, 276, p.135-141, page 136, right<br>column, paragraph of 2.2 Alu cDNA cloning | 1-23 |
| A | ***ALU WARNING: Human Alu-Sq subfamily<br>consensus sequence., Database GenBank [online],<br>Accession No. U14573.1, 1994.11.17 [Retrieved<br>on 2017.5.31], Retrieved from the Internet,<br>URL: <https://www.ncbi.nlm.nih.gov/nuccore/<br>551542>, Whole document | 1-23 |
| A | ***ALU WARNING: Human Alu-J subfamily consensus<br>sequence., Database Genbank [online], Accession<br>No.U14567.1, 1994.11.17 [Retrieved on<br>2017.5.31], Retrieved from the Internet, URL:<br><https://www.ncbi.nlm.nih.gov/nuccore/551536>,<br>Whole Document | 1-23 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8809517 B2, Ambati
- US 20140178309 A1, Ambati
- US 20150038446 A1, Ambati
- JP 2016061839 A

**Non-patent literature cited in the description**

- **HIROKO KANEKO et al.** DICER1 deficit induces Alu RNA toxicity in age-related macular degeneration. *Nature,* 2011, vol. 471, 325-330
- **VALERIA TARALLO et al.** DICER1 Loss and Alu RNA Induce Age-Related Macular Degeneration via the NLRP3 Inflammasome and MyD88. *Cell,* 2012, vol. 149 (4), 847-859
- **B.FOWLER et al.** Alu retrotransposon quantification in the retina and plasma: Mechanism-based risk assessment in age-related macular degeneration. *Acta Ophthalmologica,* 2013, vol. 91 (252

SEQUENCE LISTING

<110> Santen Pharmaceutical Co., Ltd.

<120> A kit for determining atrophic age-related maculardegeneration and a method for determining the same

<130> PH-6830-PCT

<150> JP 2016-061839
<151> 2016-03-25

<160> 15

<170> PatentIn version 3.5

<210> 1
<211> 291
<212> RNA
<213> Homo sapiens

<400> 1
ggccgggcgc gguggcucac gccuguaauc ccagcacuuu gggaggccga ggcgggugga      60

ucaccugagg ucaggaguuc gagaccagcc uggccaacau ggugaaaccc cgucucuacu     120

aaaaauacaa aaauuagccg ggcguggugg cgggcgccug uaaucccagc uacucgggag     180

gcugaggcag gagaaucgcu ugaacccggg aggcggaggu ugcagugagc cgagaucgcg     240

ccacugcacu ccagccuggg caacaagagc gaaacuccgu cucaaaaaaa a             291

<210> 2
<211> 290
<212> RNA
<213> Homo sapiens

<400> 2
ggccgggcgc gguggcucac gccuguaauc ccagcacuuu gggaggccga ggcgggagga      60

ucacuugagc ccaggaguuc gagaccagcc ugggcaacau agugaaaccc cgucucuaca     120

aaaaauacaa aaauuagccg ggcguggugg cgcgcgccug uagucccagc uacucgggag     180

gcugaggcag gaggaucgcu ugagcccggg aggucgaggc ugcagugagc cgugaucgcg     240

ccacugcacu ccagccuggg cgacagagcg agacccuguc ucaaaaaaaa             290

<210> 3
<211> 52
<212> DNA
<213> Artificial

<220>
<223> primer

<400> 3
gtgcagggtc cgaggttcac tataggtttt tttttttttt tttttttttt ag             52

<210> 4
<211> 53
<212> DNA

14

```
<213>   Artificial

<220>
<223>   primer

<400>   4
gtgcagggtc cgaggttcac tataggtttt tttttttttt tttttttttt tgt          53


<210>   5
<211>   23
<212>   DNA
<213>   Artificial

<220>
<223>   primer

<400>   5
caacatrgtg aaaccccgtc tct                                            23


<210>   6
<211>   16
<212>   DNA
<213>   Artificial

<220>
<223>   primer

<400>   6
caggagttcg agacca                                                    16


<210>   7
<211>   94
<212>   RNA
<213>   Artificial

<220>
<223>   synthetic

<400>   7
caacauggug aaaccccguc ucuacuaaaa auacaaaaau uagccgggcg ugguggcggg     60

cgccuguaau cccagcuacu cgggaggcug aggc                                94


<210>   8
<211>   19
<212>   DNA
<213>   Artificial

<220>
<223>   primer

<400>   8
gcctcagcct cccgagtag                                                 19


<210>   9
<211>   94
<212>   DNA
<213>   Artificial
```

```
<220>
<223>  synthetic

<400>  9
gcctcagcct cccgagtagc tgggattaca ggcgcccgcc accacgcccg gctaattttt          60

gtatttttag tagagacggg gtttcactat gttg                                     94


<210>  10
<211>  49
<212>  RNA
<213>  Artificial

<220>
<223>  synthetic

<400>  10
caggaguucg agaccagccu ggccaacaug gugaaacccc gucucuacu                      49


<210>  11
<211>  52
<212>  DNA
<213>  Artificial

<220>
<223>  primer

<400>  11
gtgcagggtc cgaggttcac tataggtttt tttttttttt tttttttttt ca                  52


<210>  12
<211>  16
<212>  DNA
<213>  Artificial

<220>
<223>  primer

<400>  12
gtgcagggtc cgaggt                                                         16


<210>  13
<211>  19
<212>  DNA
<213>  Artificial

<220>
<223>  primer

<400>  13
tcaccgggtg taaatcagc                                                      19


<210>  14
<211>  99
<212>  DNA
<213>  Artificial
```

```
<220>
<223>  synthetic

<400>  14
gtgcagggtc cgaggttcac tataggtttt tttttttttt tttttttttt agtagagacg          60

gggtttcacc atgttggcca ggctggtctc gaactcctg                                99


<210>  15
<211>  72
<212>  DNA
<213>  Artificial

<220>
<223>  synthetic

<400>  15
gtgcagggtc cgaggttcac tataggtttt tttttttttt tttttttttt caagctgatt          60

tacacccggt ga                                                             72
```